# EUROPEAN PATENT APPLICATION

(11) **EP 4 502 130 A1**
(43) Date of publication of application: **05.02.2025**
(21) Application number: 23189092.2
(22) Date of filing: 01.08.2023
(51) Int. Cl.: C12M 1/00, C12M 1/36

(54) **MANIFOLD UNIT FOR A BIOREACTOR DEVICE ASSEMBLY, ESPECIALLY FOR A BIOREACTOR DEVICE ASSEMBLY INCLUDING A SMALL-VOLUME BIOREACTOR**

(71) Applicant: The Automation Partnership (Cambridge) Limited, Royston, Hertfordshire SG8 5WY (GB)
(72) Inventor: Crooks, David Alan, Royston, Hertfordshire, SG8 5WY (GB); Paley, Matthew, Royston, Hertfordshire, SG8 5WY (GB); Sullivan, Mitchell, Royston, Hertfordshire, SG8 5WY (GB); Sieja, Ruben, Royston, Hertfordshire, SG8 5WY (GB)
(74) Representative: Prinz & Partner mbB

(57) **Abstract**

A manifold unit (14) for a bioreactor device assembly (10), especially for a bioreactor device assembly (10) including a small-volume bioreactor (12) for process development, comprises the following components: a plurality of mass flow controllers (16), preferably provided on a single board or block, each mass flow controller (16) having an inlet port (18) and an outlet port (20); a plurality of gas supply connectors (22) configured for connections to different gas supply lines; a plurality of gas input lines (24) connecting the gas supply connectors (22) to the inlet ports (18) of the mass flow controllers (16) according to a defined gas input distribution scheme; a plurality of gas vessel connectors (26, 28), a first gas vessel connector (26) being configured for connection to a first gassing line (30) leading to a headspace (34) of the bioreactor (12), a second gas vessel connector (28) being configured for connection to a second gassing line (32) leading to at least one sparger (36) arranged in the bioreactor (12); a plurality of gas output lines (38) connecting the outlet ports (20) of the mass flow controllers (16) to the gas vessel connectors (26, 28) according to a defined gas output distribution scheme; electrical connectors (40) configured for connections to a power supply (42) and a control unit (44); and communication and power lines (46, 48) connecting the electrical connectors (40) to the mass flow controllers (16). The components are assembled as one prefabricated unit.

## Description

The invention relates to a manifold unit for a bioreactor device assembly, especially for a bioreactor device assembly including a small-volume bioreactor for process development.

As part of an efficient biopharmaceutical development program, having a qualified scale-down model of the final manufacturing process is essential to ensure that a good understanding of critical manufacturing process parameters, which can influence product quality, is established during development.

The use of scale-down models in the development process lowers costs, improves efficiency, and reduces development times. These benefits are further improved if lower volumes and automation can be implemented. This can be achieved, for example, with the small-volume Ambr^{®} 250 HT system by Sartorius Stedim Biotech GmbH, Germany, which is a high throughput, automated bioreactor system for process development with 12 or 24 fully featured single-use 100 - 250 mL mini bioreactors. Typically, such a system has a gas flow system using a set of on/off valves with a single mass flow sensor and calibrated gas flow orifices.

It is also important that a scale-down model is a good representation of the large-scale manufacturing process to ensure that the results of any studies using the scale-down model are also representative of the large-scale manufacturing process.

One of the limitations of current low-volume small-scale models is the need for delivery of very low gas flow rates accurately. To achieve this, hitherto a pulsatile gassing technology has been implemented. However, this is not representative of larger-scale bioreactor systems, which do not use pulsatile gas supply, and may have an impact on the bioreactor physical environment, e. g. volumetric mass transfer coefficient (kLa) and shear, that cells may respond to differently when compared with the larger-scale bioreactors.

It is therefore an object of the invention to enable small-scale bioreactors, preferably in a multi-parallel arrangement, to have accurate continuous gas delivery that is more representative of technologies implemented on large-scale bioreactors.

The above problem is solved by a manifold unit according to claim 1, by a manifold device according to claim 7, by a bioreactor device assembly according to claim 8, and by a method according to claim 10. Advantageous and expedient embodiments of the invention are apparent from the respective dependent claims.

The invention provides a manifold unit for a bioreactor device assembly, especially for a bioreactor device assembly including a small-volume bioreactor for process development. The manifold unit comprises the following components: a plurality of mass flow controllers, preferably provided on a single board or block, each mass flow controller having an inlet port and an outlet port; a plurality of gas supply connectors configured for connections to different gas supply lines; a plurality of gas input lines connecting the gas supply connectors to the inlet ports of the mass flow controllers according to a defined gas input distribution scheme; a plurality of gas vessel connectors, a first gas vessel connector being configured for connection to a first gassing line leading to a headspace of the bioreactor, a second gas vessel connector being configured for connection to a second gassing line leading to at least one sparger arranged in the bioreactor; a plurality of gas output lines connecting the outlet ports of the mass flow controllers to the gas vessel connectors according to a defined gas output distribution scheme; electrical connectors configured for connections to a power supply and a control unit; and communication and power lines connecting the electrical connectors to the mass flow controllers. The components are assembled as one prefabricated unit.

In the context of this invention, "small-volume" bioreactors are bioreactors with a volume of about 10 - 500 mL, preferably 10 - 250 mL. Typical applications of such small-volume bioreactors include process development and process optimization, scale-down studies, as well as cell culture and microbial fermentation.

The mass flow controllers (MFCs) of the manifold unit are single channel devices that measure and control the flow of gas. The invention is based on the finding that, with a plurality of mass flow controllers (two or more) and further fluidic and electrical equipment in a single manifold unit, it is possible to effectively improve the handling and control of one, or several parallel, process(es) in a high-throughput format for users. The mass flow controllers enable controlled continuous gas flow for small-scale bioreactors, so that the corresponding setups and processes are more representative of corresponding larger-scale setups and processes, respectively.

In particular, the manifold unit according to the invention allows a larger range of gas flows and mixtures of gases, smoother gassing profiles, and also reduced foam accumulation in the bioreactor. With the invention, the experimental design space at the small-scale is increased, enabling the user to test conditions that more closely match those expected at the manufacturing-scale, providing greater insight into bioreactor processes and allowing users to more easily develop and qualify their scale-down model. The scale-down model can then be used with confidence in process characterisation studies as a prerequisite to scaling-up to manufacturing scale.

In summary, the invention enables improved process control, supports scalability and process characterization in a high-throughput format for users, and enables easier qualification of scale-down models, resulting in lower development costs, improved development efficiency and reduced development time.

In operation, each used gas supply connector is connected to a different source of a certain gas, such as air, O₂, N₂, CO₂ (or the respective connector is closed if not used). Preferably, the gas input distribution scheme provides that each gas supply connector is connected to the inlet ports of a set of at least two different mass flow controllers via the gas input lines. It is of course possible that one or more of the gas supply connectors is/are connected to more than two different mass flow controllers. In any event, it is thus possible to provide a certain gas to different locations or devices of the bioreactor assembly, especially to an outlet opening into the headspace of the bioreactor, to a first sparger (e. g. a macrosparger), to a second sparger (e. g. a microsparger), etc. This means that one type of gas can be supplied to the small-volume bioreactor in different ways, where each supply path can be controlled individually via a separate mass flow controller.

Matching the above-described concept of each gas supply connector being connected to the inlet ports of a set of at least two different mass flow controllers, the gas output distribution scheme of the manifold unit preferably provides that the outlet port of a first mass flow controller of each set of mass flow controllers is connected to the first gas vessel connector via the gas output lines, and that the outlet port of a second mass flow controller of each set of mass flow controllers is connected to the second gas vessel connector via the gas output lines. This means that different types of gas can be provided to each gas vessel connector in a controlled manner. For example, air and CO₂ can flow via two separate mass flow controllers to the first gas vessel connector, which is fluidically connected to e. g. the headspace of the bioreactor. It is generally possible that only one gas is supplied to a gas vessel connector at a time (with the other MFCs whose output lines lead to the same gas vessel connector being closed), or different gases can be mixed in defined ratios by the mass flow controllers so that a gas mixture is provided at the common gas vessel connector. This concept is neither limited to a certain number of different gas types nor to a certain number of gas vessel connectors. Especially, different gases or gas mixtures may be provided to three (or even more) gas vessel connectors, which are fluidically connected to the headspace of the bioreactor, a first sparger (e. g. a macrosparger), and a second sparger (e. g. a microsparger), respectively.

The manifold unit can have a vent valve, preferably provided at a gas output line leading to the first gas vessel connector, which is fluidically connected to the headspace of the bioreactor. The vent valve can be controlled by an external (or internal) pressure sensor, so that if the pressure in the headspace (overlay) is too high (for example if the biology is very active and "foams out"), then all mass flow controllers are closed and the vent valve is opened to prevent critical overpressure and potential damage to the bioreactor or the manifold unit. Control of this procedure may be provided by an internal control unit of the manifold unit itself or at the request of a separate external control unit.

In order to provide continuous, but finely adjustable individual gas flows, it is preferred that one or more of the mass flow controllers have a very high turndown ratio of more than 1000:1, preferably more than 5000:1. The turndown ratio indicates the range of flow that a mass flow controller is able to measure and control with acceptable accuracy (e. g. +/- 10 % error). In current small-scale systems the turndown ratio of a mass flow controller is typically in the order of 1:50 or 1:100, whereas for the purpose of the invention a turndown ratio of about 1:5500 is strived at, corresponding to a range of 0.1 - 550 mL/min for continuous flow. The turndown ratio is also important when a mass flow controller is operated in pulsed mode.

According to a preferred embodiment of the invention, the gas supply connectors and/or the electric connectors and/or the gas vessel connectors are provided on an interface unit of the manifold unit. An interface unit allows reliable, standardized connections at defined locations, thus significantly facilitating the handling of the manifold device. Such an interface may be formed on a detachable plate or block or the like to also facilitate servicing.

The invention also provides a manifold device, comprising a plurality of, preferably a set of 12 or 24, manifold units as defined above, assembled as one prefabricated unit. Such a combined bulk of manifold units allows controlled gas supply to a large range of separate bioreactors. Each bioreactor can operate at a different or identical set of gas flow conditions

The invention further provides a whole bioreactor device assembly, comprising at least one bioreactor, especially a small-volume bioreactor for process development, the bioreactor having a headspace and at least one sparger; at least one manifold unit as defined above, or at least one manifold device as defined above; a plurality of different gas sources supplying different gases; a plurality of gas supply lines connecting each gas source to a different one of the gas supply connectors of the manifold unit; a first gassing line connecting the first gas vessel connector of the manifold unit to the headspace of the bioreactor; a second gassing line connecting the second gas vessel connector of the manifold unit to the sparger in the bioreactor; a power supply for supplying power to the mass flow controllers; a control unit for operating the mass flow controllers; and a power line and at least one communication line connecting the power supply and the control unit to the electrical connectors of the mass flow controllers. With these components, a fully operable bioreactor device assembly is provided with the benefits of the one or more manifold units as explained above. The control unit of the manifold device, which is connected to the mass flow controllers, can be - at least partly - integrated into the hardware of the manifold unit, or it can be provided as a separate external unit.

According to an especially preferred embodiment of the invention, the bioreactor device assembly comprises a plurality of, preferably a set of 12 or 24, bioreactors and one manifold unit for each bioreactor, preferably one manifold device as defined above for each set of bioreactors.

The invention also provides a method of using the manifold unit as defined above, or the manifold device as defined above, in a bioreactor device assembly as defined above. In particular, the control unit can be configured to operate the mass flow controllers in various ways for different purposes in a flexible manner. Some important configurations are explained below.

It is recalled that continuous, fine-tuned gas flows for small-volume bioreactors are the main object of the invention. Accordingly, the control unit at least temporarily operates one or more of the mass flow controllers to provide a continuous gas flow.

However, under certain circumstances it can be advantageous to at least temporarily operate one or more of the mass flow controllers to provide a pulsed gas flow. Preferably, switching between continuous and pulsed operation modes is possible.

In order to obtain the required accuracy at low flows it may be necessary to "zero" one or more of the mass flow sensors of the mass flow controllers. However, this is not ideal for the biology if it requires constant low flow. In order to solve this problem, the control unit operates one or more of the mass flow controllers to slightly increase the gas flow before and/or after a short no-flow period in which one or more mass flow sensors of the mass flow controllers are tared. This concept, which can be described as "interleaved taring", allows a compromise between maintaining the flow and the requirement of having a short period of time where there is no flow to allow a tare. The cause of the slight increase of the gas flow before and/or after the no-flow tare is to get ahead of the flow requirement, and/or to compensate after the no-flow period.

In large-volume bioreactors usually a check valve (one-way valve) is used to prevent that liquid enters into a sparger when it is not operated. However, this is not possible in a small tube of a small-volume bioreactor. This problem can be solved by a mode of operation where one or more of the mass flow controllers (repeatedly) provide temporary gas outputs through the second gas vessel port, which is fluidically connected to the sparger in the bioreactor, to prevent liquid in the bioreactor from proceeding to the second gas vessel port through the sparger and the second gassing line. For example, this can be achieved by a "low flow" command (MFC operated to supply e. g. 0.1 mL/min) or by a "fixed duration" command (MFC operated to supply e. g. 5 mL/min for 200 ms) when the sparger is not otherwise used. This mode of operation can be activated and deactivated depending on the sparge operation requirements. Of course, such "background gassing" and similar techniques can be used with any sparger, if more than one sparger is used, e. g. a microsparger and a macrosparger; i. e. the mass flow controllers are operated to (repeatedly) provide temporary gas outputs through any gas vessel port which is fluidically connected to a sparger in the bioreactor.

Moreover, the control unit can operate one or more of the mass flow controllers to provide a temporary gas output through the second gas vessel port - or any gas vessel port that is fluidically connected to a sparger in the bioreactor - to purge the respective sparger and/or to prevent the respective sparger from becoming blocked, e. g. by cells or other objects.

If a gas mixture is needed, the control unit can operate one or more of the mass flow controllers to output a defined mixture of different gasses to the first and/or second gas vessel connectors, or to any other gas vessel connector that is fluidically connected to an outlet or a sparger in the bioreactor.

According to one aspect, the defined mixture of different gasses can be used to calibrate a gas sensor, especially an inlet gas sensor or an off-gas sensor, of the bioreactor.

The control unit can also operate one or more of the mass flow controllers to prevent a reverse gas flow through the gas output lines of the manifold unit and/or the gassing lines, in order to prevent contamination of components of the manifold unit. This means that an unused mass flow controller will automatically be closed so that no gas from any other mass flow controller, whose gas output line is linked to the gas output line of the unused mass flow controller, can pass through the unused mass flow controller in the reverse gas flow direction.

For safety reasons, the control unit can be configured to ensure that a maximum total gas output of the mass flow controllers at the first and/or second gas vessel connectors, which preferably can be specified by a user, is not exceeded, while maintaining the gas mixture ratio. This ensures that a misconfiguration, which could cause an excessive amount of gas to be output by the mass flow controllers, is prevented. While doing so, it can be ensured that the mass flow controllers limit the respective gas flows proportionally, so that the overall amount of gas is reduced, but the desired gas mixture ratio is not changed. However, other methods of preventing too high gas flows can be used, depending on requirements.

According to another aspect, the control unit can operate one or more of the mass flow controllers to purge CO₂ from the headspace of the bioreactor and/or from a liquid in the bioreactor. This is helpful to stop CO₂ dissolving into the liquid and sucking bioreactor content into the gas supply system.

The control unit of the manifold unit can be integrated into a high-level feedback loop control. Such a loop control may involve a PID controller, for example.

Further features and advantages of the invention will become apparent from the following description and from the accompanying drawings to which reference is made. In the drawings:
- Figure 1 schematically shows a manifold unit in a bioreactor device assembly including a small-volume bioreactor; and
- Figure 2 shows a diagram illustrating the concept of interleaved taring.

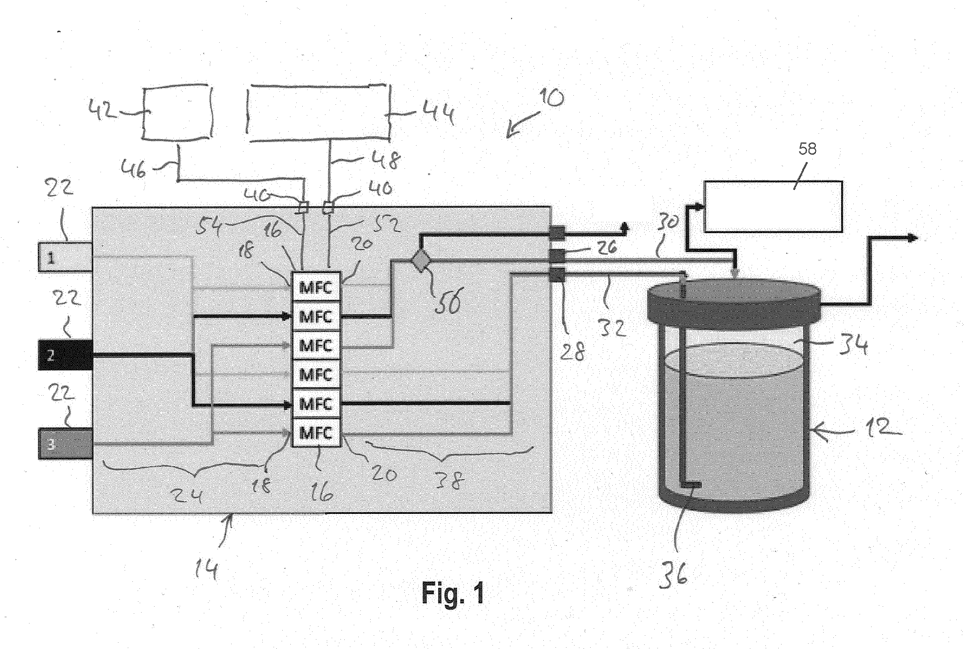

A bioreactor device assembly 10 comprising a small-volume bioreactor 12 is shown in Figure 1. The bioreactor device assembly 10 further comprises a manifold unit 14 including a plurality of mass flow controllers (MFCs) 16. In the embodiment shown in Figure 1, six separate mass flow controllers 16 are provided, each having a very high turndown ratio of more than 1000:1, preferably more than 5000: 1.

Each mass flow controller 16 has an inlet port 18 and an outlet port 20. Each inlet port 18 is connected to one of a plurality of gas supply connectors 22 via a gas input line 24. In turn, each gas supply connector 22 is connected to a different source of gas (not shown), such as air, O₂, N₂, CO₂. In the embodiment shown in Figure 1, three separate gas supply connectors 22 are provided for supplying three different types of gas.

The gas input lines 24 are configured according to a predefined gas input distribution scheme. Here, the gas input distribution scheme provides that each gas supply connector 22 is connected to the inlet ports 18 of a set of two different mass flow controllers 16. Accordingly, first type of gas can be supplied to two separate mass flow controllers 16, a second type of gas can be supplied to another two separate mass flow controllers 16, and a third type of gas can be supplied to still another two separate mass flow controllers 16. It is to be noted that the gas input distribution scheme is neither limited to three gas supply connectors 22 nor to six separate mass flow controllers 16.

Each outlet port 20 of the mass flow controllers 16 is connected to a gas output line 38. The gas output lines 38 are configured according to a predefined gas output distribution scheme. Here, the gas output distribution scheme provides that the outlet port 20 of a first mass flow controller 16 of each set of two mass flow controllers 16 is connected to a first gas vessel connector 26, and that the outlet port 20 of a second mass flow controller 16 of each set of mass flow controllers 16 is connected to a second gas vessel connector 28. This means that three different types of gas can be supplied in a controlled manner to the first gas vessel connector 26, and the same three different types of gas can also be supplied in a controlled manner to the second gas vessel connector 28. Again, it is to be noted that the gas output distribution scheme is neither limited to two gas vessel connectors 26, 28 nor to six separate mass flow controllers 16.

The first and second gas vessel connectors 26, 28 are connected to first and second gassing lines, 30, 32, respectively. The first gassing line 30 leads to the headspace 34 of the bioreactor 12 above the liquid level. The second gassing line 32 leads to a sparger 36 arranged in the bioreactor 12. According to the same concept, a further gas vessel connector may be provided which could lead to another sparger in the bioreactor 12 via a third gassing line, for example.

It is to be noted that the gas input lines 24 and/or the gas output lines 38 can be formed as flexible hoses or rigid tubing. According to a special alternative, the manifold unit 14 includes a solid block and the gas input lines 24 and/or the gas output lines 38 are drilled into the solid block to make the manifold unit 14 very compact.

The manifold unit 14 further comprises electrical connectors 40 configured for connection to an external power supply 42 and a control unit 44 via power lines 46 and communication lines 48, respectively. The mass flow controllers 16 are connected to the respective electrical connectors 40 via power lines 52 and communication lines 54 of the manifold unit 14.

All components of the manifold unit 14, except for the external power supply 42, the control unit 44, the power lines 46 and the communication lines 48, are assembled as one prefabricated unit. However, the control unit 44 can also be - at least partly - provided as an integral part of the manifold device 14. Alternatively, it is be provided as a separate external unit.

The gas supply connectors 22 and/or the electric connectors 40 and/or the gas vessel connectors 26, 28 can be provided on one or more interface units.

A further component of the manifold unit 14 is a vent valve 56 provided at a gas output line 38 leading to the first gas vessel connector 26, which is fluidically connected to the headspace 34 of the bioreactor 34. The vent valve 56 is indirectly controlled by an external or internal pressure sensor 58 which measures the pressure in the headspace 34. Both the vent valve 56 and the pressure sensor 58 are connected to the control unit 44.

The control unit 44 is configured to operate the mass flow controllers 16 as described further above.

Although a bioreactor device assembly 10 comprising only a single bioreactor 12 and a single manifold unit 14 is shown in Figure 1, the bioreactor device assembly 10 can comprise a plurality of, preferably a set of 12 or 24, bioreactors 12 with one manifold unit 14 for each bioreactor 12. The plurality of manifold units 14 can be combined to form one compact manifold device.

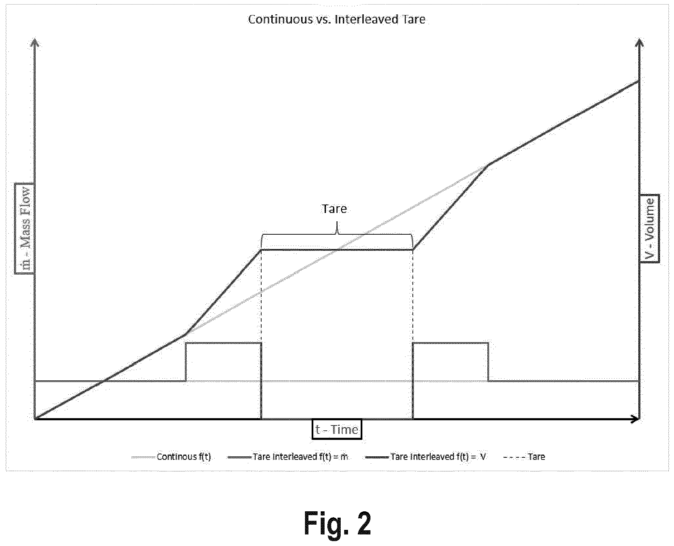

Figure 2 shows a diagram illustrating the concept of interleaved taring in situations when it is necessary to "zero" one or more of the mass flow sensors of the mass flow controllers to obtain the required accuracy at low flows. The diagram shows the cumulative gas volume at a constant flow rate (mass flow) over time vs. an interleaved arrangement. With a constant flow rate (continuous) the cumulated volume rises linearly With the interleaved arrangement the flow rate is decreased, e. g. from 0.1 mL/min to 0 mL/min for about one minute, with a short time of e. g. 0.2 mL/min before and after the no-flow period, so that the average flow rate over a longer time period is maintained, e. g. at 0.1 mL/min.

Another feature that can be used in the bioreactor device assembly 10 with the manifold unit 14 relates to the pressure in the headspace 34 of the bioreactor 12. Measuring headspace pressure while having continuous flow generates an elevated pressure reading. That is caused by the dynamic pressure of the flow which is digitally compensated for. The dynamic pressures are characterised, and those values are not represented in the vessel's headspace pressure readings.

Apart from the main fields of application, which involve small-volume bioreactors, the above-described invention can also be used in applications with large-volume bioreactors.

### List of Reference Signs

- 10: bioreactor device assembly
- 12: bioreactor
- 14: manifold unit
- 16: mass flow controller (MFC)
- 18: inlet port
- 20: outlet port
- 22: gas supply connector
- 24: gas input line
- 26: first gas vessel connector
- 28: second gas vessel connector
- 30: first gassing line
- 32: second gassing line
- 34: headspace
- 36: sparger
- 38: gas output line
- 40: electrical connector
- 42: power supply
- 44: control unit
- 46: power line
- 48: power line
- 52: power line
- 54: communication line
- 56: vent valve
- 58: pressure sensor

## Claims

1. A manifold unit (14) for a bioreactor device assembly (10), especially for a bioreactor device assembly (10) including a small-volume bioreactor (12) for process development, the manifold unit (14) comprising the following components:
a plurality of mass flow controllers (16), preferably provided on a single board or block, each mass flow controller (16) having an inlet port (18) and an outlet port (20);
a plurality of gas supply connectors (22) configured for connections to different gas supply lines;
a plurality of gas input lines (24) connecting the gas supply connectors (22) to the inlet ports (18) of the mass flow controllers (16) according to a defined gas input distribution scheme;
a plurality of gas vessel connectors (26, 28), a first gas vessel connector (26) being configured for connection to a first gassing line (30) leading to a headspace (34) of the bioreactor (12), a second gas vessel connector (28) being configured for connection to a second gassing line (32) leading to at least one sparger (36) arranged in the bioreactor (12);
a plurality of gas output lines (38) connecting the outlet ports (20) of the mass flow controllers (16) to the gas vessel connectors (26, 28) according to a defined gas output distribution scheme;
electrical connectors (40) configured for connections to a power supply (42) and a control unit (44); and
communication and power lines (46, 48) connecting the electrical connectors (40) to the mass flow controllers (16);
wherein the components are assembled as one prefabricated unit.

2. The manifold unit (14) according to claim 1, **characterised in that** the gas input distribution scheme provides that each gas supply connector (22) is connected to the inlet ports (18) of a set of at least two different mass flow controllers (16) via the gas input lines (24).

3. The manifold unit (14) according to claim 2, **characterised in that** the gas output distribution scheme provides that the outlet port (20) of a first mass flow controller (16) of each set of mass flow controllers (16) is connected to the first gas vessel connector (26) via the gas output lines (38), and that the outlet port (20) of a second mass flow controller (16) of each set of mass flow controllers (16) is connected to the second gas vessel connector (28) via the gas output lines (38).

4. The manifold unit (14) according to any of the preceding claims, **characterised by** a vent valve (56) provided at a gas output line (38) leading to the first gas vessel connector (26).

5. The manifold unit (14) according to any of the preceding claims, **characterised in that** one or more of the mass flow controllers (16) have a turndown ratio of more than 1000:1, preferably more than 5000:1.

6. The manifold unit (14) according to any of the preceding claims, **characterised in that** the gas supply connectors (22) and/or the electric connectors (40) and/or the gas vessel connectors (26, 28) are provided on an interface unit.

7. A manifold device, comprising a plurality of, preferably a set of 12 or 24, manifold units (14) according to any of the preceding claims, assembled as one prefabricated unit.

8. A bioreactor device assembly (10), comprising:
at least one bioreactor (12), especially a small-volume bioreactor (12) for process development, the bioreactor (12) having a headspace (34) and at least one sparger (36);
at least one manifold unit (14) according to any of claims 1 to 6, or at least one manifold device according to claim 7;
a plurality of different gas sources supplying different gases;
a plurality of gas supply lines connecting each gas source to a different one of the gas supply connectors (22) of the manifold unit (14);
a first gassing line (30) connecting the first gas vessel connector (26) of the manifold unit (14) to the headspace of the bioreactor (12);
a second gassing line (32) connecting the second gas vessel connector (28) of the manifold unit (14) to the sparger (36) in the bioreactor (12);
a power supply (42) for supplying power to the mass flow controllers (16);
a control unit (44) for operating the mass flow controllers (16); and
a power line (52) and at least one communication line (54) connecting the power supply (42) and the control unit (44) to the electrical connectors (40) of the mass flow controllers (16).

9. The bioreactor device assembly (10) according to claim 8, **characterised in that** the bioreactor device assembly (10) comprises a plurality of, preferably a set of 12 or 24, bioreactors (12) and one manifold unit (14) for each bioreactor (12), preferably one manifold device according to claim 7 for each set of bioreactors (12).

10. A method of using the manifold unit (14) according to any of claims 1 to 6 or the manifold device according to claim 7 in a bioreactor device assembly (10) according to claim 8 or 9.

11. The method according to claim 10, **characterised in that** the control unit at least temporarily operates one or more of the mass flow controllers (16) to provide a continuous gas flow.

12. The method according to claim 10 or 11, **characterised in that** the control unit (44) at least temporarily operates one or more of the mass flow controllers (16) to provide a pulsed gas flow.

13. The method according to any of claims 10 to 12, **characterised in that** the control unit (44) operates one or more of the mass flow controllers (16) to slightly increase a gas flow before and/or after a short no-flow period in which one or more mass flow sensors of the mass flow controllers (16) are tared.

14. The method according to any of claims 10 to 13, **characterised in that** the control unit (44) operates one or more of the mass flow controllers (16) to provide temporary gas outputs through the second gas vessel port (28) to prevent liquid in the bioreactor (12) from proceeding to the second gas vessel port (28) through the sparger (36) and the second gassing line (32).

15. The method according to any of claims 10 to 14, **characterised in that** the control unit (44) operates one or more of the mass flow controllers (16) to provide a temporary gas output through the second gas vessel port (28) to purge the sparger (36) and/or to prevent the sparger (36) from becoming blocked.

16. The method according to any of claims 10 to 15, **characterised in that** the control unit (44) operates one or more of the mass flow controllers (16) to output a defined mixture of different gasses to the first and/or second gas vessel connectors (26, 28).

17. The method unit according to claim 16, **characterised in that** the defined mixture of different gasses is used to calibrate a gas sensor, especially an inlet gas sensor or an off-gas sensor, of the bioreactor (12).

18. The method according to any of claims 10 to 17, **characterised in that** the control unit (44) operates one or more of the mass flow controllers (16) to prevent a reverse gas flow through the gas output lines (38) of the manifold unit (14) and/or the gassing lines (30, 32).

19. The method according to any of claims 10 to 18, **characterised in that** the control unit (44) ensures that a maximum total gas output of the mass flow controllers (16) at the first and/or second gas vessel connectors (26, 28), which can preferably be specified by a user, is not exceeded.

20. The method according to any of claims 10 to 19, **characterised in that** the control unit (44) operates one or more of the mass flow controllers (16) to purge CO₂ from the headspace (34) of the bioreactor (12) and/or from a liquid in the bioreactor (12).

21. The method according to any of claims 10 to 20, **characterised in that** the control unit (44) is integrated into a high-level feedback loop control.
